(19) Europäisches Patentamt
European Patent Office
Office européen des brevets

(11) Veröffentlichungsnummer: **0 029 982**
**A1**

(12) # EUROPÄISCHE PATENTANMELDUNG

(21) Anmeldenummer: **80107306.5**

(22) Anmeldetag: **24.11.80**

(51) Int. Cl.³: **C 07 D 217/24**
A 61 K 31/64

(30) Priorität: **01.12.79 DE 2948472**

(43) Veröffentlichungstag der Anmeldung:
**10.06.81 Patentblatt 81/23**

(84) Benannte Vertragsstaaten:
**AT BE CH DE FR GB IT LI NL**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Hitzel, Volker, Dr.**
**Kantstrasse 1b**
**D-6238 Hofheim am Taunus(DE)**

(72) Erfinder: **Weyer, Rudi, Dr.**
**Johann-Strauss-Strasse 43**
**D-6233 Kelkheim (Taunus)(DE)**

(72) Erfinder: **Geisen, Karl, Dr.**
**Jahnstrasse 43**
**D-6000 Frankfurt am Main(DE)**

(72) Erfinder: **Regitz, Günter, Dr.**
**Dachbergstrasse 68**
**D-6232 Bad Soden am Taunus(DE)**

(54) **Benzolsulfonylharnstoffe, Verfahren zu ihrer Herstellung, pharmazeutische Präparate auf Basis dieser Verbindungen sowie ihre Verwendung.**

(57) Sulfonylharnstoffe der Formel

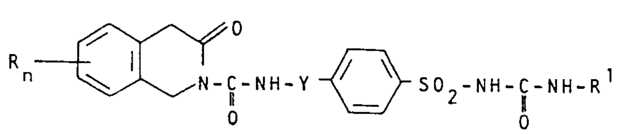

worin n, R, R¹ und Y die angegebenen Bedeutungen haben, sowie deren physiologisch verträgliche Salze, pharmazeutische Präparate auf Basis dieser Verbindungen und ihre Verwendung bei der Behandlung der Diabetes.

EP 0 029 982 A1

HOECHST AKTIENGESELLSCHAFT HOE 79/F 327 Dr.D/sch

# Benzolsulfonylharnstoffe, Verfahren zu ihrer Herstellung, pharmazeutische Präparate auf Basis dieser Verbindungen sowie ihre Verwendung

Die Erfindung betrifft Sulfonylharnstoffe der Formel

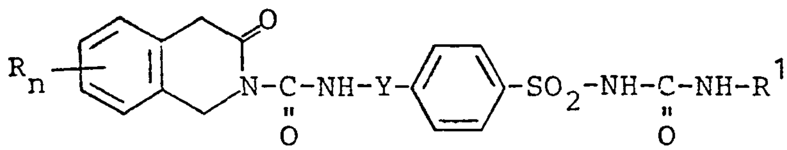

die als Substanz oder in Form ihrer Salze blutzuckersenkende Eigenschaften besitzen und sich durch starke und langanhaltende Senkung des Blutzuckerspiegels auszeichnen.

In der Formel bedeuten

n 1 oder 2

R Wasserstoff, Alkyl mit 1 bis 4 Kohlenstoffatomen, Alkoxy mit 1 bis 4 Kohlenstoffatomen oder Halogen, wobei, falls n 2 ist, die R gleich oder verschieden sein können,

Y Alkylen mit 2 bis 3 C-Atomen,

$R^1$ Alkyl von 3 bis 8 C-Atomen, Cycloalkyl, Alkylcycloalkyl, Dialkylcycloalkyl, Cycloalkylalkyl, Cycloalkenyl, Alkylcycloalkenyl mit jeweils 5 bis 9 C-Atomen, Methylcyclopentylmethyl Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl.

In der allgemeinen Formel bedeutet R vorzugsweise Wasserstoff. Y bedeutet vorzugsweise $-CH_2-CH_2-$, $-CH(CH_3)-CH_2-$, wobei die $-CH_2-CH_2-$Gruppe besonders bevorzugt ist. $R^1$ ist vorzugsweise Butyl, Cyclohexyl, Methylcyclohexyl, besonders bevorzugt ist 4-Methyl-cyclohexyl. Als bicyclische Reste kommen beispielsweise in Frage: Bicyclo/2.2.1/heptyl, Bicyclo-

/2.2.1/heptylmethyl sowie die entsprechenden ungesättigten Reste und der Bicyclo/2.2.2/octylrest.

Gegenstand der Erfindung sind ferner Verfahren zur Herstellung dieser Sulfonylharnstoffe, pharmazeutische Präparate, die diese enthalten oder aus ihnen bestehen sowie ihre Verwendung zur Behandlung des Diabetes.

Die Verfahren zur Herstellung sind dadurch gekennzeichnet, daß man

a) mit der Gruppe

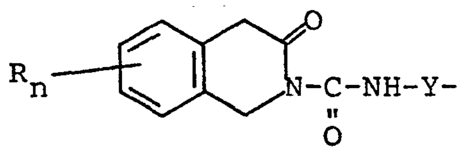

in 4-Stellung substituierte Benzolsulfonyl-isocyanate, -carbaminsäureester, -thiolcarbaminsäureester, -harnstoffe, -semicarbazide oder -semicarbazone mit einem Amin $R^1-NH_2$ oder dessen Salzen umsetzt oder Sulfonamide der Formel

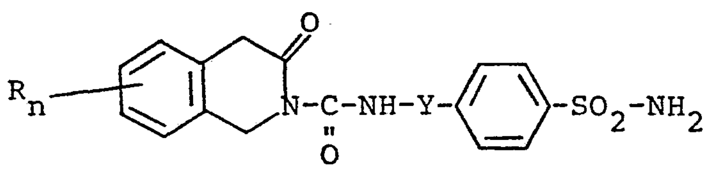

oder deren Salze mit $R^1$-substituierten Isocyanaten, Carbaminsäureestern, Thiolcarbaminsäureestern, Carbaminsäurehalogeniden oder Harnstoffe umsetzt,

b) entsprechend substituierte Benzolsulfonyl-isoharnstoffäther, -isothioharnstoffäther, -parabansäuren oder -halogenameisensäureamidine spaltet,

c) in

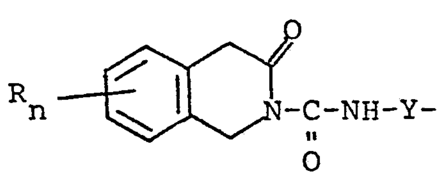

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffen der Formel

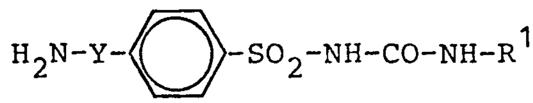

gegebenenfalls stufenweise den Rest

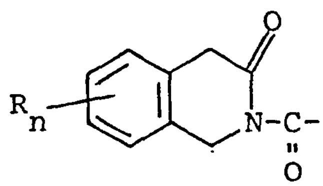

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^1$-N'-hydroxyharnstoff umsetzt
und die Reaktionsprodukte gegebenenfalls zur Salzbildung mit alkalischen Mitteln behandelt.

Die erwähnten Benzolsulfonyl-carbaminsäureester bzw. -thiolcarbaminsäureester können in der Alkoholkomponente einen Alkylrest oder einen Arylrest oder auch einen heterocyclischen Rest aufweisen. Da dieser Rest bei der Reaktion abgespalten wird, hat seine chemische Konstitution keinen Einfluß auf den Charakter des Endproduktes und kann deshalb in weiten Grenzen variiert werden. Das gleiche gilt für die N-$R^1$-substituierten Carbaminsäureester bzw. die entsprechenden Thiolcarbaminsäureester.

Als Carbaminsäurehalogenide eignen sich in erster Linie die Chloride.

Die als Ausgangsstoffe des Verfahrens infrage kommenden Benzolsulfonylharnstoffe können an der der Sulfonylgruppe abgewandten Seite des Harnstoffmoleküls unsubstituiert oder ein- oder insbesondere zweifach substituiert sein. Da diese Substituenten bei der Reaktion mit Aminen abgespalten werden, kann ihr Charakter in weiten Grenzen variiert werden. Neben alkyl-, aryl-, acyl- oder heterocyclisch substituierten Benzolsulfonylharnstoffen kann man auch Benzolsulfonylcarbamoylimidazole und ähnliche Verbindungen oder Bisbenzolsulfonylharnstoffe, die an einem der Stickstoffatome noch einen weiteren Substituenten z.B. Methyl, tragen können, verwenden. Man kann beispielsweise derartige Bis-(benzolsulfonyl)-harnstoffe oder auch N-Benzolsulfonyl-N'-acylharnstoffe mit $R^1$-substituierten Aminen behandeln und die erhaltenen Salze auf erhöhte Temperaturen, insbesondere solche oberhalb 100°C, erhitzen.

Weiterhin ist es möglich, von $R^1$-substituierten Harnstoffen auszugehen oder von solchen $R^1$-substituierten Harnstoffen, die am freien Stickstoffatom noch ein- oder insbesondere zweifach substituiert sind und diese mit

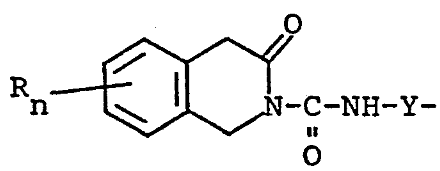

in 4-Stellung substituierten Benzolsulfonamiden umzusetzen. Als solche Ausgangsstoffe kommen beispielsweise infrage N-Cyclohexyl-harnstoff, die entsprechenden N'-Acetyl, N'-Nitro, N'-Cyclohexyl, N',N'-Diphenyl- (wobei die beiden Phenylreste auch substituiert sowie direkt oder auch über ein Brückenglied wie $-CH_2-$, -NH-, -O- oder -S- miteinander verbunden sein können), N'-Methyl-N'-phenyl-, N',N'-Dicyclohexylharnstoffe sowie Cyclohexyl-carbamoyl-imidazole, -pyrazole oder -triazole sowie solche der genannten Ver-

0029982

bindungen, die anstelle des Cyclohexyls einen anderen im Bereich der Definition für $R^1$ liegenden Substituenten tragen.

Die Spaltung der als Ausgangsstoffe genannten Benzolsulfonylparabänsäuren, -isoharnstoffäther, -isothioharnstoffäther oder -halogenameisensäureamidine erfolgt zweckmäßig durch alkalische Hydrolyse. Isoharnstoffäther können auch in einem sauren Medium mit gutem Erfolg gespalten werden.

Der Ersatz des Schwefelatoms in der Thioharnstoffgruppierung von entsprechend substituierten Benzolsulfonylthioharnstoffen durch ein Sauerstoffatom kann in bekannter Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten ausgeführt werden. Die Thioharnstoffe können auch entschwefelt werden durch Behandlung mit Phosgen oder Phosphorpentachlorid. Als Zwischenstufe erhaltene Chlorameisensäureamidine bzw. Carbodiimide können durch geeignete Maßnahmen wie Verseifen oder Anlagerung von Wasser in die Benzolsulfonylharnstoffe überführt werden.

Die Oxydation von Benzolsulfinyl- bzw. Benzolsulfenylharnstoffen erfolgt nach an sich bekannter Methode, vorzugsweise mit Oxydationsmitteln wie Permanganat oder Wasserstoffperoxid.

Die Acylierung der Sulfonylharnstoffe gemäß Verfahren e) kann mit reaktiven Derivaten der Säure

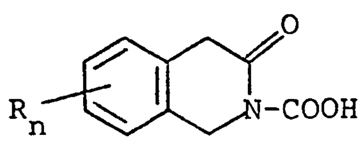

wie beispielsweise Halogeniden oder Urethanen erfolgen.

Als Sulfonyl- bzw. Sulfinylhalogenide gemäß Verfahren f) eignen sich insbesondere die Chloride. Als saures Kondensationsmittel kann man beispielsweise Thionylchlorid oder Polyphosphorsäure einsetzen.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen.

Die Synthese der 3-Oxo-1.2.3.4-tetrahydro-isochinoline erfolgt nach bereits beschriebenen Verfahren (vergl. z.B. US-PS 3 796 717) oder kann in Analogie dazu durchgeführt werden.

Die Ausführungsformen des Verfahrens gemäß der Erfindung können im allgemeinen hinsichtlich der Reaktionsbedingungen weitgehend variiert und den jeweiligen Verhältnissen angepaßt werden. Beispielsweise können die Umsetzungen in Abwesenheit oder Anwesenheit von Lösungsmitteln, bei Raumtemperatur oder bei erhöhter Temperatur durchgeführt werden.

Je nach dem Charakter der Ausgangsstoffe kann das eine oder andere der beschriebenen Verfahren in einzelnen Fällen einen gewünschten individuellen Benzolsulfonylharnstoff nur in geringen Ausbeuten liefern oder zu dessen Synthese nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die erhaltenen Verbindungen können durch Umfällen und/oder Umkristallisieren gereinigt werden. Die Reinigung kann auch erfolgen, indem man die Substanz aus einem kristallinen (Alkali-)Salz in einem geeigneten Lösungsmittel in Freiheit setzt.

Die erfindungsgemäßen Verbindungen zeichnen sich durch wertvolle pharmakologische Eigenschaften, insbesondere

blutzuckersenkende, aus. Sie eignen sich daher als Arzneimittel, insbesondere als Antidiabetika.

Die blutzuckersenkende Wirkung der beschriebenen Benzolsulfonylharnstoffe kann dadurch festgestellt werden, daß man sie als freie Verbindungen oder in Form der Natriumsalze in Dosen von 10 mg oder 2 mg/kg an normal ernährte Kaninchen verfüttert und den Blutzuckerwert nach der bekannten Methode von Hagedorn-Jensen oder mit einem Autoanalyzer über eine längere Zeitdauer ermittelt.

Die Bestimmung der blutzuckersenkenden Wirkung kann aber auch mit geringeren Dosen oder nach anderen bekannten Methoden erfolgen.

Die folgenden Verbindungen I bis IV wurden in Dosierungen von 2 mg/kg Kaninchen verabreicht und die Blutzuckerwerte wurden mit einem Autoanalyzer über eine längere Zeitdauer ermittelt. Die hierbei gemessene Blutzuckersenkung ist in der nachfolgenden Tabelle in % nach .... Stunden angegeben.

I N-(4-<2-(3-Oxo-1,2,3,4-tetrahydroisochinolin-2-yl-carboxamido)-äthyl>-benzolsulfonyl)-N'-butyl-harnstoff

II N-(4-<2-(3-Oxo-1,2,3,4-tetrahydroisochinolin-2-yl-carboxamido)-äthyl>-benzolsulfonyl)-N'-cyclopentyl-harnstoff

III N-(4-<2-(3-Oxo-1,2,3,4-tetrahydroisochinolin-2-yl-carboxamido)-äthyl>-benzolsulfonyl)-N'-(4-methyl-cyclohexyl)-harnstoff

IV N-(4-<2-(3-Oxo-1,2,3,4-tetrahydroisochinolin-2-yl-carboxamido)-äthyl>-benzolsulfonyl)-N'-($\Delta^3$-cyclohexenyl)-harnstoff

Tabelle

| Verbindung | Blutzuckersenkung am Kaninchen nach oraler Verabreichung von 2 mg/kg in % nach | | | | |
|---|---|---|---|---|---|
| | 1 | 3 | 6 | 24 | 48 Stunden |
| I | 32 | 29 | 37 | 17 | 0 |
| II | 33 | 42 | 41 | 40 | 8 |
| III | 34 | 35 | 41 | 38 | 0 |
| IV | 35 | 36 | 35 | 27 | 0 |

Die erfindungsgemäßen Acylureidoalkylbenzolsulfonylharnstoffe zeichnen sich durch eine starke und langanhaltende blutzuckersenkende Wirkung aus.

Die Eigenschaften der Verbindungen erlauben es, in der Therapie des Diabetes mellitus mit so geringen Dosen auszukommen, daß das Präparat nur die verminderte Ansprechbarkeit des Pankreas auf einen erhöhten Blutzuckerspiegel wieder normalisiert.

Benzolsulfonylharnstoffe mit Ureidoalkylrest sind schon mehrfach beschrieben worden (DE-PS 14 43 911, DE-AS 16 70 700, DE-PS 16 18 389, DE-PS 22 38 870). Es war nicht zu erwarten, daß die erfindungsgemäßen Verbindungen sich durch die oben erwähnten günstigen Eigenschaften auszeichnen.

Die erfindungsgemäßen Sulfonylharnstoffe sollen vorzugsweise zur Herstellung von oral verabreichbaren Präparaten zur Behandlung des Diabetes mellitus dienen. Sie können als solche oder in Form ihrer Salze bzw. in Gegenwart von Stoffen, die zu einer Salzbildung führen, appliziert werden. Zur Salzbildung können beispielsweise alkalische Mittel wie Alkali- oder Erdalkalihydroxyde, -carbonate oder -bicarbonate herangezogen werden. Die Präparate können neben dem Sulfonylharnstoff bzw. dessen Salz auch noch andere Wirkstoffe enthalten.

Ein Präparat, das die beschriebenen Benzolsulfonylharnstoffe als Wirkstoff enthält, z.B. eine Tablette oder ein Pulver mit oder ohne Zusätze, ist zweckmäßig in eine geeignet dosierte Form gebracht. Als Dosis ist dabei eine solche zu wählen, die der Wirksamkeit des verwendeten Benzolsulfonylharnstoffs und dem gewünschten Effekt angepaßt ist. Zweckmäßig beträgt die Dosierung je Einheit etwa 0,1 bis 10 mg, vorzugsweise 0,5 bis 2 mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemäßen Sulfonylharnstoffe verwendet werden können. Sie sollen jedoch nicht eine Einschränkung des Erfindungsgegenstandes darstellen.

Beispiel 1:

N-(4-⟨2-(3-Oxo-1,2,3,4-tetrahydro-isochinolin-2-yl-carboxamido)-äthyl⟩-benzolsulfonyl)-N'-cyclohexyl-harnstoff

1,87 g 4-(2-⟨3-Oxo-1,2,3,4-tetrahydro-isochinolin-2-yl-carboxamido⟩-äthyl)-benzolsulfonamid (Schmp. 188 - 190°C, hergestellt durch Reaktion von 3-Oxo-1,2,3,4-tetrahydro-isochinolin-2-yl-(N-2-phenyl-äthyl)-carboxamid ⟨Schmp. 82 - 84°C, hergestellt aus 3-Oxo-1,2,3,4-tetrahydro-isochinolin und Phenyl-äthylisocyanat⟩ mit Chlorsulfonsäure und Umsetzung des erhaltenen Sulfochlorids mit Ammoniak) werden in 25 ml Aceton und 2,5 ml 2N Natronlauge gelöst. Nach kurzem Rühren fällt das Natriumsalz aus. Man kühlt auf 0°C ab, tropft unter Rühren eine Lösung von 0,73 g Cyclohexylisocyanat in wenig Aceton zu und rührt 4 Stunden nach, wobei man auf Raumtemperatur erwärmen läßt. Der entstandene Niederschlag wird mit wenig Wasser in Lösung gebracht, die Lösung filtriert und mit 2N Salzsäure angesäuert. Der ausgefällte N-(4-⟨2-(3-Oxo-1,2,3,4-tetrahydro-isochinolin-2-yl-carboxamido)-äthyl⟩-benzolsulfonyl)-N'-cyclohexyl-harnstoff wird abgesaugt und aus Äthanol-Dimethylformamid umkristallisiert. Er schmilzt bei 187 - 189°C.

In analoger Weise erhält man den

N-(4-⟨2-(3-Oxo-1,2,3,4-tetrahydroisochinolin-2-yl-carboxamido)-äthyl⟩-benzolsulfonyl)-N′-(4-methyl-cyclohexyl)-harnstoff
vom Schmp. 158 - 160°C (aus Äthanol)

N-(4-⟨2-(3-Oxo-1,2,3,4-tetrahydroisochinolin-2-yl-carboxamido)-äthyl⟩-benzolsulfonyl)-N′-(4-äthyl-cyclohexyl)-harnstoff
vom Schmp. 176 - 178°C (aus Äthanol)

N-(4-⟨2-(3-Oxo-1,2,3,4-tetrahydroisochinolin-2-yl-carboxamido)-äthyl⟩-benzolsulfonyl)-N′-($\Delta^3$-cyclohexenyl)-harnstoff
vom Schmp. 169 - 171°C (aus Äthanol)

N-(4-⟨2-(3-Oxo-1,2,3,4-tetrahydroisochinolin-2-yl-carboxamido)-äthyl⟩-benzolsulfonyl)-N′-butyl-harnstoff
vom Schmp. 137 - 139°C (aus Äthanol)

N-(4-⟨2-(3-Oxo-1,2,3,4-tetrahydroisochinolin-2-yl-carboxamido)-äthyl⟩-benzolsulfonyl)-N′-propyl-harnstoff
vom Schmp. 85°C (Zersetzung) (umgefällt über das Ammonium-Salz)

N-(4-⟨2-(3-Oxo-1,2,3,4-tetrahydroisochinolin-2-yl-carboxamido)-äthyl⟩-benzolsulfonyl)-N′-benzyl-harnstoff
vom Schmp. 109 - 110°C (aus Äthanol)

N-(4-⟨2-(3-Oxo-1,2,3,4-tetrahydroisochinolin-2-yl-carboxamido)-äthyl⟩-benzolsulfonyl)-N'-cyclopentyl-harnstoff
vom Schmp. 172 - 173°C (aus Äthanol)

N-(4-⟨2-(3-Oxo-1,2,3,4-tetrahydro-isochinolin-2-yl-carboxamido)-äthyl⟩-benzolsulfonyl)-N'-cycloheptyl-harnstoff
vom Schmp. 177 - 179 °C (aus Äthanol)

Beispiel 2:

N-(4-⟨2-(3-Oxo-1,2,3,4-tetrahydro-isochinolin-2-yl-carboxamido)-äthyl⟩-benzolsulfonyl)-N'-hexyl-harnstoff

2,8 g 4-(2-⟨3-Oxo-1,2,3,4-tetrahydro-isochinolin-2-yl-carboxamido⟩-äthyl)-benzolsulfonamid (Schmp. und Herstellung siehe Beispiel 1) werden in 50 ml Aceton und 25 ml Dioxan zusammen mit 2,0 g gemahlener Pottasche 3 Stunden unter Rückfluß gerührt. Nach kurzem Abkühlen tropft man 1,05 g n-Hexylisocyanat zu und rührt weitere 4 Stunden unter Rückfluß nach. Die Suspension wird eingedampft, der Rückstand mit Wasser in Lösung gebracht, die Lösung mit 2N Salzsäure angesäuert und mit Chloroform extrahiert. Nach dem Trocknen wird eingeengt und aus Essigsäureäthylester umkristallisiert. Der so hergestellte N-(4-⟨2-(3-Oxo-1,2,3,4-tetrahydro-isochinolin-2-yl-carboxamido)-äthyl⟩-benzolsulfonyl)-N'-hexyl-harnstoff schmilzt bei 99 - 100°C.

In analoger Weise erhält man den

N-(4-⟨2-(3-Oxo-1,2,3,4-tetrahydro-isochinolin-2-yl-carboxamido)-äthyl⟩-benzolsulfonyl)-N′-bicyclo/2.2.1/-hept-2-yl-methyl-harnstoff vom Schmp. 138 - 140°C (mit Äther verrieben)

N-(4-⟨2-(3-Oxo-1,2,3,4-tetrahydro-isochinolin-2-yl-carboxamido)-äthyl⟩-benzolsulfonyl)-N′-(3-methylcyclopentyl)-harnstoff vom Schmp. 143 - 145°C (aus Aceton mit Diisopropyläther gefällt)

N-(4-⟨2-(3-Oxo-1,2,3,4-tetrahydro-isochinolin-2-yl-carboxamido)-äthyl⟩-benzolsulfonyl)-N′-cyclopentylmethyl-harnstoff vom Schmp. 155 - 157°C (aus Äthanol)

Beispiel 3:

N-(4-⟨2-(3-Oxo-1,2,3,4-tetrahydro-isochinolin-2-yl-carboxamido)-äthyl⟩-benzolsulfonyl)-N′-cyclohexyl-harnstoff

2,94 g 3-Oxo-1,2,3,4-tetrahydro-isochinolin werden in 50 ml abs. Toluol mit 0,96 g Natriumhydrid-Suspension (55%ig in Paraffin) sechs Stunden unter Rückfluß gerührt. Man kühlt ab und setzt 20 ml einer 25%-igen Phosgen-Lösung in Toluol zu. Anschließend rührt man zwei Stunden bei 45°C nach, läßt abkühlen und verdünnt mit 50 ml Toluol. Nach dem Filtrieren engt man im Vakuum ein.

3,14 g des so erhaltenen 3-Oxo-1,2,3,4-tetrahydro-isochinolin-2-carbamoylchlorids werden in 50 ml Methylenchlorid gelöst und zu einer bei Raumtemperatur gerührten Suspension von 4,75 g N-(4-⟨2-Aminoäthyl⟩-benzolsulfonyl)-N′-cyclohexyl-harnstoff in 75 ml Tetrahydrofuran und 4,2 ml Triäthylamin getropft. Man rührt vier Stunden bei Raumtemperatur nach und engt die Suspension im Vakuum ein. Der Rückstand wird aus verdünnter Ammoniaklösung mit verdünnter Salzsäure umgefällt und nach dem Absaugen und Trocknen aus Äthanol-Dimethylformamid umkristallisiert. Der auf diese Weise hergestellte N-(4-⟨2-(3-Oxo-1,2,3,4-tetrahydro-isochinolin-2-yl-carboxamido)-äthyl⟩-benzolsulfonyl)-N′-cyclohexyl-harnstoff schmilzt bei 186 - 188°C und zeigt keine Schmelzpunktdepression mit der nach Beispiel 1 synthetisierten Substanz.

Beispiel 4:

**N-(4-⟨2-(3-Oxo-1,2,3,4-tetrahydro-isochinolin-2-yl-carboxamido)-äthyl⟩-benzolsulfonyl)-N'-cyclohexyl-harnstoff**

1,52 g N-Cyclohexyl-harnstoff werden in 50 ml abs. Tetrahydrofuran gelöst und mit 0,6 g Natriumhydrid-Suspension (55%ig in Paraffin) versetzt. Man rührt drei Stunden unter Rückfluß, kühlt auf 20°C ab und tropft eine Lösung von 3,92 g 4-(2-⟨3-Oxo-1,2,3,4-tetrahydro-isochinolin-2-yl-carboxamido⟩-äthyl)-benzolsulfochlorid (Fp. 115 - 116°C) in 50 ml abs. Tetrahydrofuran zu und rührt weitere drei Stunden unter Rückfluß nach. Die gesamte Mischung wird im Vakuum eingedampft und der Rückstand in Wasser gelöst. Nach dem Filtrieren und Ansäuern wird zweimal mit Chloroform extrahiert. Die Chloroformphase wird getrocknet und eingeengt und der Rückstand aus Äthanol-Dimethylformamid umkristallisiert. Der so erhaltene N-(4-⟨2-(3-Oxo-1,2,3,4-tetrahydro-isochinolin-2-yl-carboxamido)-äthyl⟩-benzolsulfonyl)-N'-cyclohexyl-harnstoff schmilzt bei 185 - 188°C und zeigt im Mischschmelzpunkt mit einer auf andere Weise hergestellten Probe keine Depression.

**Beispiel 5:**

**N-(4-⟨2-(3-Oxo-1,2,3,4-tetrahydroisochinolin-2-yl-carboxamido)-äthyl⟩-benzolsulfonyl)-N'-(4-chlor-cyclohexyl)-harnstoff**

1,2 g N-(4-⟨2-(3-Oxo-1,2,3,4-tetrahydroisochinolin-2-yl-carboxamido)-äthyl⟩-benzolsulfonyl)-carbamidsäuremethylester (vom Schmp. 187 - 189°C, hergestellt aus dem entsprechenden Sulfonamid, Natriumhydrid und Chlorameisensäuremethylester in Tetrahydrofuran) werden mit 0,75 g 4-Chlor-cyclohexylamin in 30 ml Dioxan unter Rückfluß gerührt. Die Lösung wird im Vakuum eingedampft und der Rückstand in Wasser aufgenommen. Nach dem Filtrieren unter Zusatz von Aktivkohle wird mit 2 N Salzsäure angesäuert und die saure Lösung mit Essigester extrahiert. Die über Natriumsulfat getrocknete Essigesterphase wird eingeengt und der als Rückstand verbleibende N-(4-⟨2-(3-Oxo-1,2,3,4-tetrahydroisochinolin-2-yl-carboxamido)-äthyl⟩-benzolsulfonyl)-N'-(4-chlor-cyclohexyl)-harnstoff wird aus wenig Essigester umkristallisiert. Er schmilzt bei 178 - 180°C.

Patentansprüche:

1. Sulfonylharnstoffe der Formel

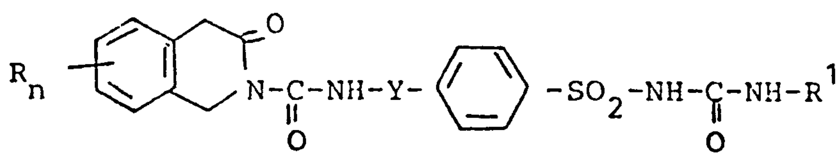

in welcher bedeuten:

n 1 oder 2

R Wasserstoff, Alkyl mit 1 bis 4 Kohlenstoffatomen, Alkoxy mit 1 bis 4 Kohlenstoffatomen oder Halogen, wobei, falls n 2 ist, die R gleich oder verschieden sein können,

Y Alkylen mit 2 bis 3 C-Atomen,

$R^1$ Alkyl von 3 bis 8 C-Atomen, Cycloalkyl, Alkylcycloalkyl, Dialkylcycloalkyl, Cycloalkylalkyl, Cycloalkenyl, Alkylcycloalkenyl mit jeweils 5 bis 9 C-Atomen, Methylcyclopentylmethyl Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl.

und deren physiologisch verträglichen Salze.

2. Verfahren zur Herstellung von Sulfonylharnstoffen gemäß Anspruch 1, dadurch gekennzeichnet, daß man

a) mit der Gruppe

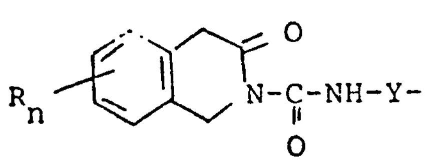

in 4-Stellung substituierte Benzolsulfonyl-isocyanate, -carbaminsäureester, -thiolcarbaminsäureester, -harnstoffe, -semicarbazide oder -semicarbazone mit einem Amin $R^1-NH_2$ oder dessen Salzen umsetzt oder Sulfonamide der Formel

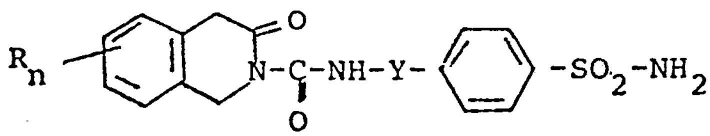

oder deren Salze mit $R^1$-substituierten Isocyanaten, Carbaminsäureestern, Thiolcarbaminsäureestern, Carbaminsäurehalogeniden oder Harnstoffe umsetzt,

b) entsprechend substituierte Benzolsulfonyl-isoharnstoffäther, -isothioharnstoffäther, -parabansäuren oder -halogenameisensäureamidine spaltet,

c) in

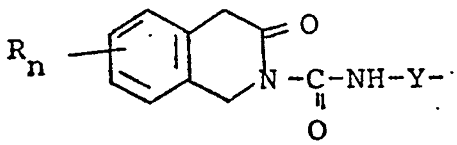

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffen der Formel

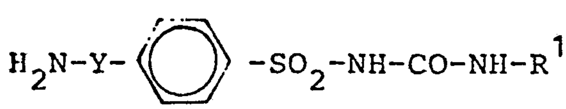

gegebenenfalls stufenweise den Reste

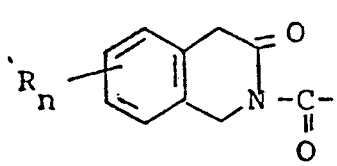

einführt,

f) entsrechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^1$-N'-hydroxyharnstoff umsetzt und die Reaktionsprodukte gegebenenfalls zur Salzbildung mit alkalischen Mitteln behandelt.

3. Arzneimittel auf Basis eines Sulfonylharnstoffs gemäß Anspruch 1 oder eines seiner Salze.

4. Verwendung eines Sulfonylharnstoffs gemäß Anspruch 1 oder eines seiner Salze bei der Bekämpfung von Diabetes.

5. Verfahren zur Herstellung eines Arzneimittels gemäß Anspruch 3, dadurch gekennzeichnet, daß man einen Sulfonylharnstoff der im Anspruch 1 angegebenen Formel oder eines seiner Salze in eine geeignete Applikationsform bringt.

6. Verfahren zur Senkung des Blutzuckerspiegels bei der Behandlung des Diabetes, dadurch gekennzeichnet, daß man eine wirksame Menge einer Verbindung gemäß Anspruch 1 verabreicht.

Patentanspruch für Österreich:

Verfahren zur Herstellung von Sulfonylharnstoffen der Formel

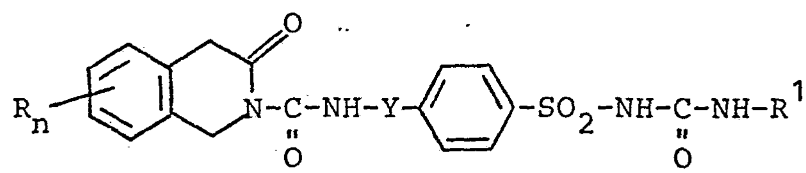

in welcher bedeuten:

n 1 oder 2

R Wasserstoff, Alkyl mit 1 bis 4 Kohlenstoffatomen, Alkoxy mit 1 bis 4 Kohlenstoffatomen oder Halogen, wobei, falls n 2 ist, die R gleich oder verschieden sein können,

Y Alkylen mit 2 bis 3 C-Atomen,

$R^1$ Alkyl von 3 bis 8 C-Atomen, Cycloalkyl, Alkylcycloalkyl, Dialkylcycloalkyl, Cycloalkylalkyl, Cycloalkenyl, Alkylcycloalkenyl mit jeweils 5 bis 9 C-Atomen, Methylcyclopentylmethyl Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl

und von deren physiologisch verträglichen Salzen, dadurch gekennzeichnet, daß man

a) mit der Gruppe

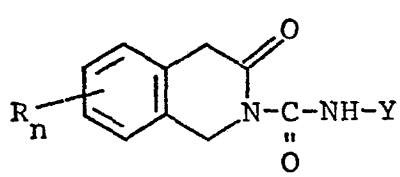

in 4-Stellung substituierte Benzolsulfonyl-isocyanate, -carbaminsäureester, -thiolcarbaminsäureester, -harnstoffe, -semicarbazide oder -semicarbazone mit einem Amin $R^1-NH_2$ oder dessen Salzen umsetzt oder Sulfonamide der Formel

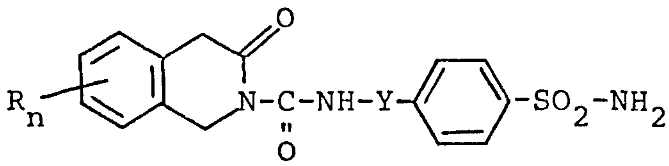

oder deren Salze mit $R^1$-substituierten Isocyanaten, Carbaminsäureestern, Thiolcarbaminsäureestern, Carbaminsäurehalogeniden oder Harnstoffe umsetzt,

b) entsprechend substituierte Benzolsulfonyl-isoharnstoffäther, -isothioharnstoffäther, -parabansäuren oder -halogenameisensäureamidine spaltet,

c) in

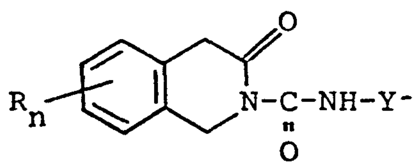

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffen der Formel

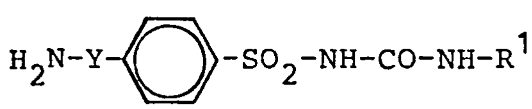

gegebenenfalls stufenweise den Rest

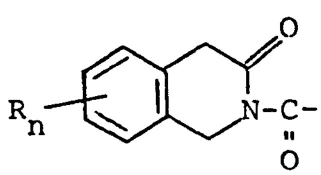

einführt,

f) entsprechend substituierte Benzolsulfonylhalogenide mit $R^1$-substituierten Harnstoffen oder deren Alkalisalzen

umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^1$-N'-hydroxyharnstoff umsetzt
und die Reaktionsprodukte gegebenenfalls zur Salzbildung mit alkalischen Mitteln behandelt.

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

**0029982**
Nummer der Anmeldung

EP 80107306.5

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int Cl.) |
|---|---|---|---|
| | DE - A1 - 2 621 958 (HOECHST)<br>+ Ansprüche; Seite 10, 3. Absatz - Seite 14, erster Absatz +<br>---- | 1-3,5 | C 07 D 217/24<br>A 61 K 31/64 |

RECHERCHIERTE SACHGEBIETE (Int Cl.)

C 07 D 213/00
C 07 D 217/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5
Unvollständig recherchierte Patentansprüche: -
Nicht recherchierte Patentansprüche: 4,6
Grund für die Beschrankung der Recherche:

Verfahren zur therapeutischen Behandlung des menschlischen oder tierischen Körpers, Art. 52(4) EPÜ

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 11-02-1981 | TENGLER |

EPA Form 1505.1 06.78